# EUROPEAN PATENT SPECIFICATION

(11) **EP 1 263 429 B1**
(45) Date of publication and mention of the grant of the patent: **14.07.2004**
(21) Application number: 01917979.5
(22) Date of filing: 19.03.2001
(51) Int. Cl.: A61K 31/22, A61P 31/12, A61P 31/18

(54) **ANTIVIRAL THERAPY**
ANTIVIRALE THERAPIE
THERAPIE ANTIVIRALE

(30) Priority: 17.03.2000 EP 00200991
(43) Date of publication of application: 11.12.2002
(73) Proprietor: Universiteit Utrecht, 3584 CG Utrecht (NL); Universitair Medisch Centrum Utrecht, 3584 CX Utrecht (NL)
(72) Inventor: NOTTET, Johannes, Servatius, Leonardus, Maria, NL-1095 DX Amsterdam (NL)
(74) Representative: Prins, Adrianus Willem, Mr. Ir.
(86) International application number: PCT/NL2001/000222
(87) International publication number: WO 2001/068086

(56) References cited:
- EP-A- 1 064 940
- WO-A-98/28292
- WO-A-98/29382
- WO-A-99/30721

## Description

The invention relates to the field of antiviral agents, and more specifically to antiviral therapy.

One of the great paradoxes of medicine is that the simplest of organisms are the most difficult to control. While great progress has been made in controlling more complex organisms, for example bacteria, with hundreds of different antibacterial pharmaceutical compositions or antibiotics, there are very few pharmaceutical compositions intended or adapted for antiviral therapy that are of proven effectiveness. The major drawback in developing antiviral agents has been an inability to distinguish viral replicative mechanisms from host replicative processes. Nevertheless, progress has been made over the past two decades in discovering molecules necessary for virus replication, in characterising them mechanistically, and in developing antiviral agents to inhibit them (for review see Hirsch et al., In: Fields Virology, Chapter 15, Lippincot-Raven Publishers, 1996). Well known antiviral agents are for example amantadine and rimandatine and other anti-influenza agents, acyclovir, gangcyclovir and related agents, foscarnet and other anti-herpesvirus agents, ribavirine and various antiretroviral agents as discussed below.

Progress and understanding in the field of antiretroviral therapy in the past 3 years has been dramatic (for review see Hammer and Yeni. AIDS, 12:S181-S188, 1998). Progress has been fuelled by three major advances. First, increasing knowledge of disease pathogenesis has provided underpinnings for current therapeutic rationale. The proliferative nature of the viral replicative process (10¹⁰ virions produced and destroyed each day), the rapid viral turnover (virion plasma half-life of 6 h or less), and the recognition of second and third phases of viral decay under the influence of potent antiretroviral therapy resulting from the presence of longer-lived cell reservoirs has guided the current principles of antiretroviral therapy. The second advance has been the widening array of therapeutic choices represented by the increasing numbers of agents available to patients and clinicians. Third, the availability of increasingly sophisticated patient monitoring techniques, such as viral load determinations, has simultaneously provided the tools for dissecting HIV disease pathogenesis and monitoring the effects of treatment in affected individuals. Taken together, these developments have led to the generally accepted principle that potent combination regimens (also called highly active antiviral therapy or HAART) designed to drive and maintain plasma HIV-RNA concentration below the limits of detection of currently available assays are the treatments of choice.

However, a number of practical limitations to this idealised approach have increasingly been recognised. These include: the variability of initial virologic response according to the disease stage, particularly the high rate of failure in those with advanced HIV infection; the challenge of patient adherence to complex regimens; drug failure and the threat of multidrug resistance; the lack of predictably effective salvage therapies; the emergence of longer-term toxicities to the protease inhibitor class of compounds; and the sharpening division between populations of the world related to cost and access to effective agents.

In several countries there are 11 agents approved for the treatment of HIV infection and the reasonable expectation is that the total will rise to 15 shortly. These agents are either HIV reverse transcriptase inhibitors of the nucleoside, non-nucleoside, and nucleotide subclasses or members of the HIV-protease inhibitor class. Although the simple calculation of the number of two-, three- and four-drug combinations would suggest that the regimen choices for initial and alternative therapies are vast, in reality they are much more limited as a result of cross-resistance, toxicities, tolerance, drug or food interactions and other practical considerations. Thus, although it is true that the options for initial potent, combination regimens are increasing, when one considers the limitations on subsequent regimens conferred by the initial choice, one realises the restricted options for long-term virologic suppression that currently exist.

In areas where drug access is not a problem, the current recommended standard for initial therapy is a potent *in vivo* protease inhibitor combined with two nucleoside analogs with the first alternative being a non-nucleoside reverse transcriptease inhibitor in combination with two nucleoside analogs. However, the emergence of drug resistance during treatment and its association with treatment failure have been described with nearly all of the antiretroviral agents in use or in development. Therefore, resistance testing might be thought to logically assist with the choice of alternative treatment in the setting of treatment failure and assist with the choice of initial therapy when primary drug resistance is suspected. However, there are many questions that need to be answered before resistance testing (either genotypic or phenotypic) becomes accepted as a routine clinical tool. In what setting and to what extent this technology will improve decision making is not clear and drug resistance is only one of a number of reasons for treatment failure. Resistance testing results are most reflective of the selective pressure of the current drug that might emerge quickly on a new regimen. Further, one cannot always deduce the phenotypic susceptibility of a viral strain from its genotype because of assay sensitivity and resistance mutational interactions. Cross-resistance, particularly to protease inhibitors, may also be a dynamic process in which viruses are "primed" by mutations selected on a previous therapy to develop resistance more quickly when exposed to a new member of the same drug class.

Failure of a particular antiretroviral drug regimen may be defined clinically, immunologically or virologically. Increasingly, for individuals on their initial drug combination, a strict definition of failure is being applied, that is, detectable viremia following previous suppression below the detection below the detection limit of the assay being employed. With the advent of plasma HIV-RNA assays with detection limits at the approximate 50 copies/ml range, this has raised the question of whether a confirmed rise above this threshold should trigger a treatment change given the still limited therapeutic armamentarium.

The advances and the limitations of the currently available antiretroviral agents make it clear that new agents and combinations are urgently needed. On the immediate horizon is the promise of widespread availability of four new agents: abacavir(a nucleoside analog reverse transcriptase inhibitor), efavirenz (a non-nucleoside reverse transcriptase inhibitor), adefovir dipivoxil (a nucleotide reverse transcriptase inhibitor), and amprenavir (a protease inhibitor). These agents will carry with them an increasing number of choices for patients and clinicians but are most likely to benefit antiretroviral-naive or minimally drug-experienced individuals only. Their role in "salvage" regimens is currently under investigation but the potential for cross-resistance with the currently approved agents may well limit their effectiveness in this circumstance.

In conclusion, a next wave of drug development is needed that involves new classes of antiviral agents. Other potential anti-viral agents effective against viral targets are needed to broaden the therapeutic possibilities of viral therapy.

The invention provides use of a compound of the general formula 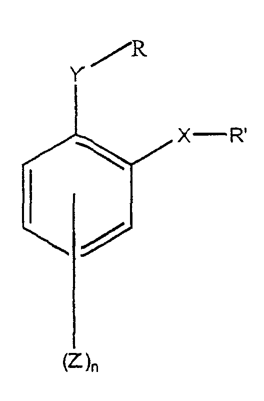 wherein X and Y are independently O, S, SO, SO₂, SO₃, but preferably O,S, SO₂; wherein n is 0, 1, 2, 3, 4, but preferably 1 or 2; wherein R, R' are independently H with the proviso that when R is H, R' is not H, a C₁-C₁₀, branched or unbranched, substituted or unsubstituted (preferably the substitute is one or more of halogen or CF₃), saturated or (poly)unsaturated, (cyclo)alkyl, alkene, alkyn, (cyclo)aryl, aryl(cyclo)alkyl, (cyclo)alkylaryl, alkoxyaryl, alkoxyalkene, alkoxyalkyne, enyne, diene, diyne or alkoxyalkyl, preferably selected from the group consisting of H, CH₃, CF₃, CH₂Cl, CH₂Br, CH₂CH₃, (CH₂)₂CH₃, (CH₂)₃CH_{3,} (CH₂)₄CH₃, (CH₂)₅CH₃, (CH₂)₆CH₃, CH(CH₃)₂, C(CH₃)_{3,} CH=C=CH₂, (CH₂)₂O(CH₂)₃CH₃, CH₂HC=CH(CH₂)₃CH₃, CH₂C≡C(CH₂)₃CH₃, CH₂C≡C(CH₂)₂CH₃, CH₂C≡C-CH₂CH₃, CH₂C≡C-CH₃ and CH₂C≡CH and isomers or homologues thereof; and wherein R' is R, preferably selected from the group consisting of H, CH₃,; and wherein R or R' may contain ether linkages or carbonyl or thiocarbonyl functions attached to the ring structure such as ring-(C=O/S)-R/R'
and Z is independently R, R', XR, XR', YR or YR'
or a functional equivalent thereof for the production of a pharmaceutical composition for the treatment of a viral infection.

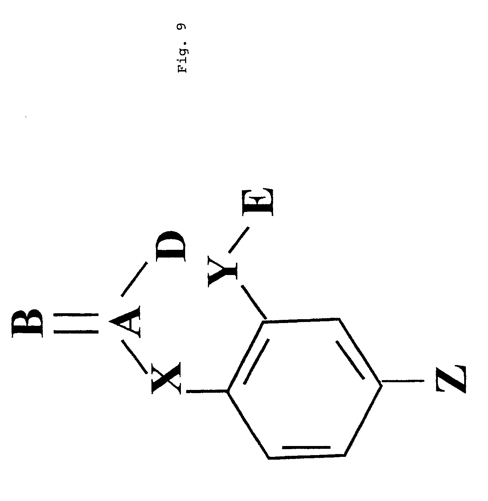

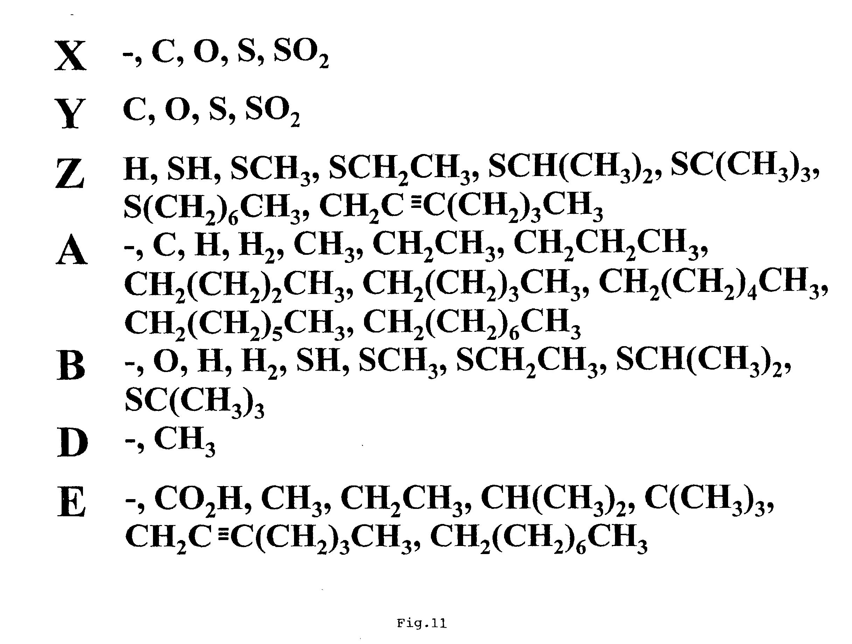

The present invention also provides use of at least one compound or mixture of comp ounds of the general formula 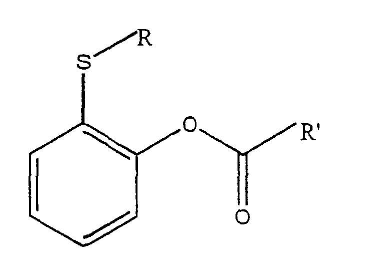 or a functional equivalent or pharmaceutically acceptable salt or hydrate thereof for the production of a pharmaceutical composition for the treatment of a viral infection. Replacements or substitutions of said general formula for example comprise replacing S with O, Se or Te, and/or additionally substituting the ring with one or more side groups such as R or R'. Functional equivalent compounds, as regard to anti-viral activity (shown as tested) are for example found among those of figure 9. Preferred are the combinations as indicated in figure 11 whereby Z = H, Y = S, E = CH₂CEC(CH₂)₃CH₃, X = O, B = -, D= -, and A is either CH₃, CH₂CH₃, CH₂CH₂CH₃, CH₂(CH₂)₂CH₃, CH₂(CH₂)₃CH₃, CH₂(CH₂)₄CH₃, CH₂(CH₂)₅CH₃, or CH₂(CH₂)₆CH₃ and the combinations in letters whereby Z = H, Y = S, E = C(CH₃)₃, X = O, A = C, B = O, and D = CH₃ and the combinations whereby Z = H, Y = S, E = C(CH₃)₃, X = -, A = C, B = O, and D = CH₃ and the combinations whereby Z = SC(CH₃)₃, Y = -, E= -, X = O, A = C, B = O, and D = CH₃ and the combinations whereby Z = SC(CH₃)₃, Y = -, E= -, X = -, A = C, B = O, and D = CH₃ and the combinations whereby Y = -, E = -, Z = SCH₂CEC(CH₂)₃CH₃, X = O, B = -, D= -, and A is either CH₃, CH₂CH₃, CH₂CH₂CH₃, CH₂(CH₂)₂CH₃, CH₂(CH₂)₃CH₃, CH₂(CH₂)₄CH₃, CH₂(CH₂)₅CH₃, or CH₂(CH₂)₆CH₃.

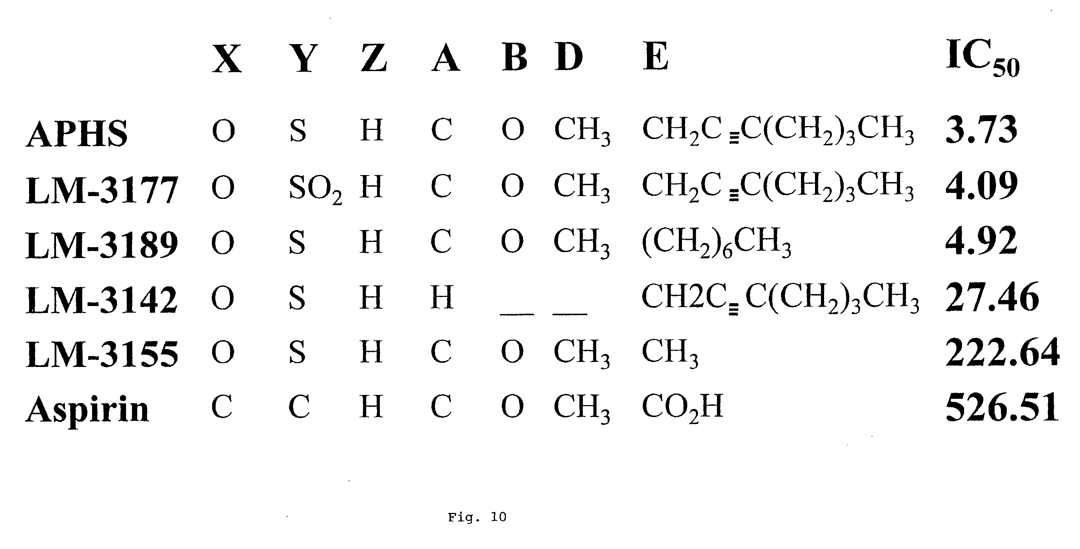

Also, the results of compounds of figure 9, shown in figure 10, shows a benzene ring comprising an ester, an ether or a hydroxyl group and comprising a lipophilic side chain being involved in anti-viral activity. Furthermore, LM-3155 is about 100-fold less efficient in inhibiting viral replication showing that the size of Y-R in the general formula or functionally equivalent compound as provided by the invention generally should be long. By reducing the size of Y-R in the general formula the compound becomes more hydrophilic. Thus, Y-R should be long in order to increase the lipophilic properties of the compound and therefore the anti-viral activity. An alternative is to keep the size of Y-R short and to add long groups at place Z in the general formula of the compound. In addition, although the anti-viral activity of LM-3142 is about 10-fold lower this finding suggests that the ester group is not absolutely necessary for its anti-viral activity. The reason that LM-3142 has a lower anti-viral activity is likely due to its decreased lipophilicity. Increasing its lipophilicity otherwise will again increase its anti-viral activity. Furthermore, because of the acetylester of APHS the compound hydrolyzes quickly and therefore the lipophilicity is reduced. Therefore, alternatives are provided whereby the acetylester is replaced by less easily hydrolysing side groups such as by an ethylether, propylether, butylether, pentylether, hexylether, or heptylether. The half lives of these functionally equivalent compounds is much longer and therefore a better anti-viral activity is provided.

A particular useful compound is identified in Table 3 provided herewith, especially a compound identified as c2, c3, c5 or c7, or a derivative thereof. Side group D (see figure 9) is preferably substituted with an electron withdrawal group, useful are groups such as CF₃, NO₂ or CN.

Compounds of said general formula are for example known from Arnoldi et al., J. Chem. Soc., Perkin Trans. 1 (1993), 12:1359-1366; from Poirier et al., Sulfur Lett. (1998) 10:167-173; and from Ohtsuka et al., Chem. Pharm. Bull. (1983) 31:443-453. Furthermore, it is known from Kalgutar et al., Science 280:1268-1270, (1998) and WO 98/29382 that several compounds of said general formula covalently inactivate cyclo-oxygenase-2 (COX-2) and are selective inhibitors of prostaglandin endoperoxidase-2 and that a pharmaceutical composition comprising such compound may be useful for providing pain-relief, such as in the prophylaxis or therapeutic treatment of inflammatory responses such as oedema, fever, algesia, neuromuscular pain, headache, cancer pain or arthritic pain.

Surprisingly, however, it is now found that a pharmaceutical composition comprising said compound is useful in anti-viral therapy. Not wishing to be bound by theory it is herein assumed that a compound of said general formula or a functional equivalent thereof antagonises activities of transcription factors such as AP-1, STAT and NF-κB, assumedly with the effect that viral functions such as virus transcription and/or viral gene expression are functionally inhibited, as for example can be detected by testing the effect of such compound on viral promotor activity (see e.g. figure 1). Alternatively, the effect on viral protein expression is detected by testing viral protein production in cell culture (see e.g. figures 2, 4a and 4b).

A preferred embodiment provides use according to the invention of a compound of the general formula as above or a pharmaceutically acceptable salt or hydrate thereof wherein R is H, CF₃ or a C1-C10 (but due to its increase lipophilicity preferably a C4-C10, branched or unbranched, substituted or unsubstituted (preferably the substitute is a halogen), saturated or (poly)unsaturated, (cyclo)alkyl, alkene, alkyn, (cyclo)aryl, aryl(cyclo)alkyl, (cyclo)alkylaryl, alkoxyaryl, alkoxyalkene, alkoxyalkyne, enyne, diene, diyne or alkoxyalkyl, preferably selected from the group consisting of H, CH₃, CH₂CH₃, (CH₂)₂CH₃, (CH₂)₃CH₃, (CH₂)₄CH₃, (CH₂)₅CH₃, (CH₂)₆CH₃, CH(CH₃)₂, C(CH₃)₃, CH=C=CH₂, (CH₂)₂O(CH₂)₃CH₃, CH₂HC=CH(CH₂)₃CH₃, CH₂C≡C(CH₂)₃CH₃, CH₂C≡C(CH₂)₂CH₃, CH₂C≡C-CH₂CH₃, CH₂C≡C-CH₃ AND CH₂C≡CH and isomers or homologues thereof; and wherein R' is R, preferably selected from the group consisting of H, CH₃, CF₃, CH₂Cl and CH₂Br.

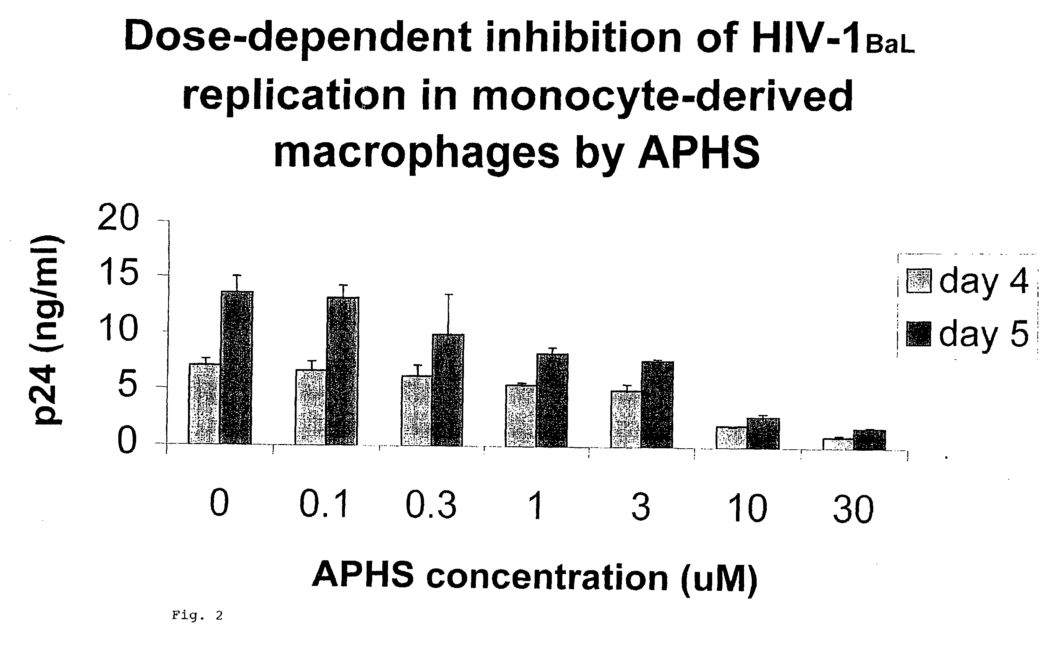

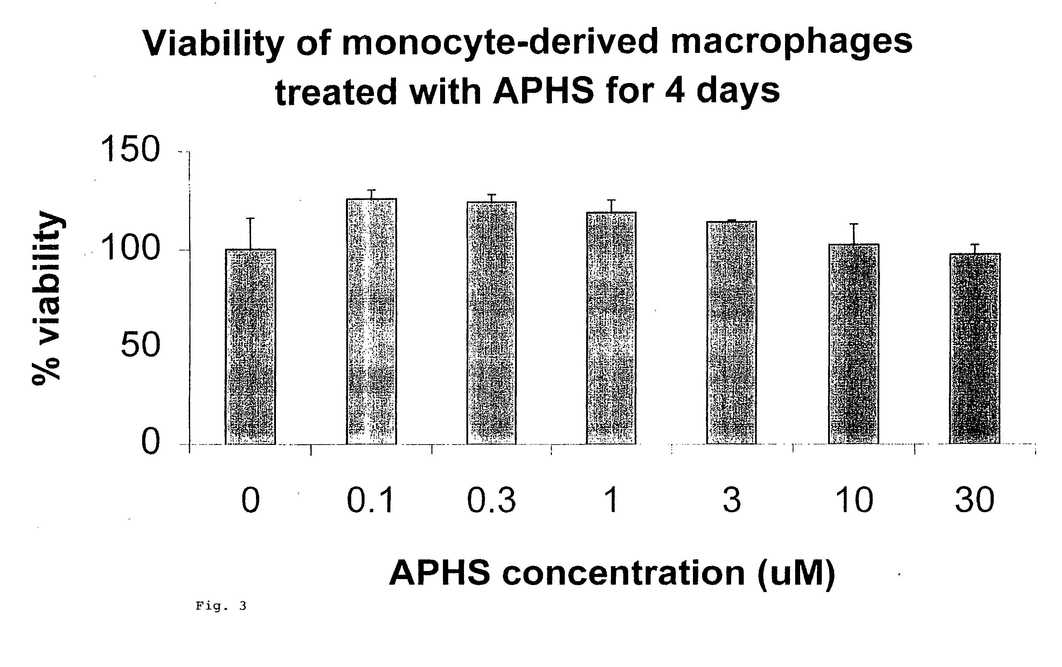

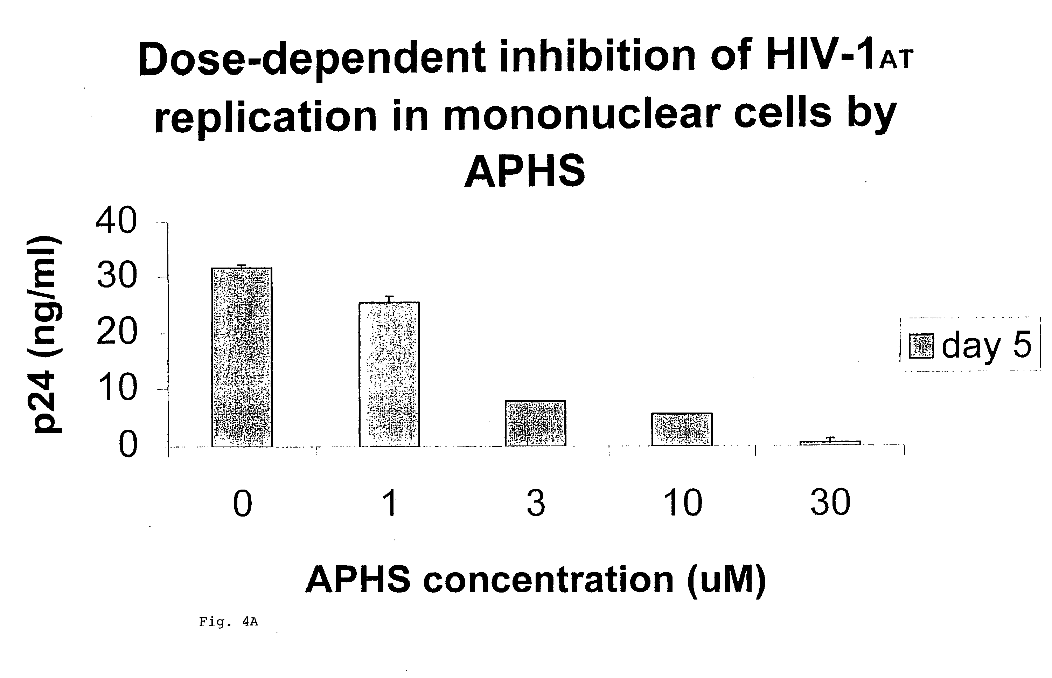

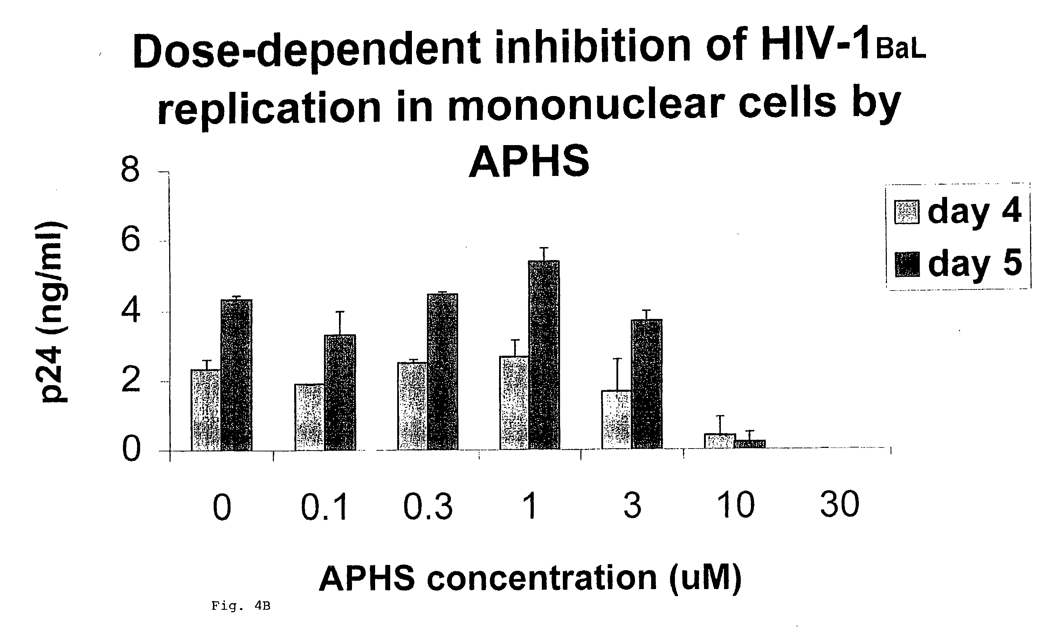

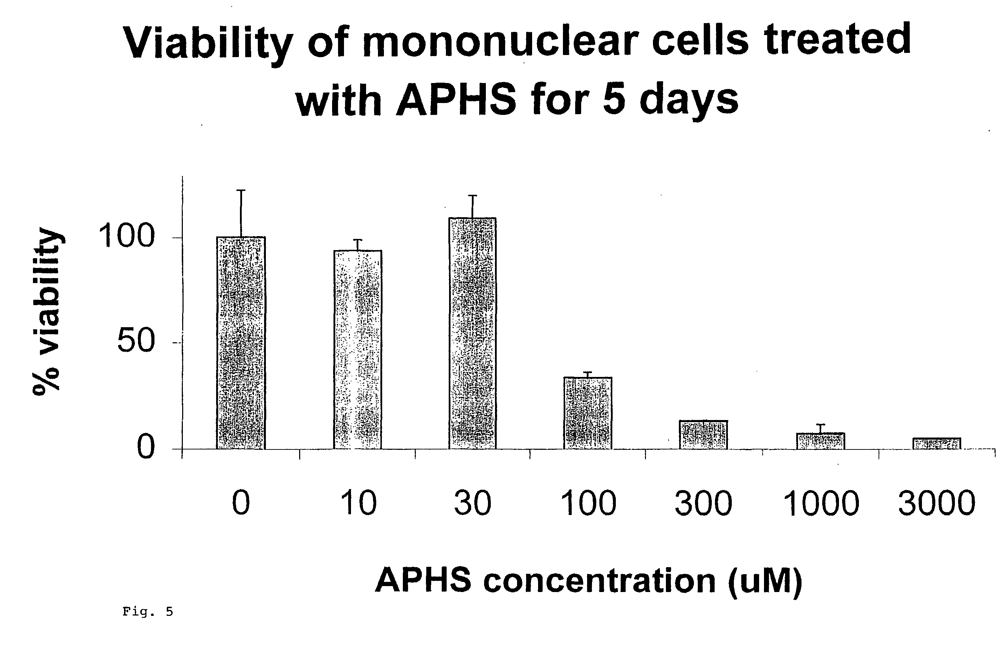

The present invention also provides a pharmaceutical composition intended and adapted for the treatment of a viral infection (herein also called an antiviral agent) comprising at least one compound or a mixture of compounds according to said general formula and a pharmaceutically acceptable carrier of diluent. In order to use a compound according to said general formula or a pharmaceutically acceptable salt or hydrate thereof in therapy, it will normally be formulated into a pharmaceutical composition in accordance with standard pharmaceutical practice. This invention, therefore, also relates to a pharmaceutical composition for the treatment of a viral infection comprising an effective amount of a compound according to said general formula, or a pharmaceutically acceptable salt or hydrate thereof, and pharmaceutically acceptable carrier or diluent. A pharmaceutical composition as provided by the invention allows treatment in a conveniently wide therapeutic window, toxicity for cells, as for example evaluated on the level of cell-viability as shown in figures 3 and 5, is not or only little detected in ranges (see figures 2, 4a and 4b) where significant anti-viral activity is found.

In a preferred embodiment, the invention provides a use according to the invention wherein said viral infection is caused by a virus at least partly resistant against treatment with another antiviral agent such as for example shown in the detailed description wherein said use is provided with an infection that is caused by a retrovirus at least partly resistant against treatment with another antiviral agent such as a reverse transcriptase inhibitor or protease inhibitor.

In a preferred embodiment, the invention provides use according to the invention wherein said viral infection comprises a retroviral infection. Such a retroviral infection can for example comprise a leukemia virus infection, such as caused by bovine leukemia virus or human T-cell-leukemia virus. Other retroviral infections known in the art are for example ovine lentivirus infections or spumaretrovirus infections. Also, such a retroviral infection can comprise an infection with a recombinant retrovirus which is for example constructed for use in gene therapy. Preferably, the invention provides use according to the invention wherein said retroviral infection is caused by a immunodeficiency virus such as human or simian immunodeficiency virus (HIV or SIV). As an example, HIV-1 infection of T-cells and macrophages is mediated by CD4 and the recently discovered chemokine receptors such as CCR-5, CXCR-4, CCR2b and CCR-3. After binding of HIV-1 gp120 to these receptors fusion of viral and cellular membranes occurs resulting in the release of the viral preintegration complex into the cytoplasm. Subsequently the matrix domain of the HIV-1 gag protein mediates the translocation of the HIV-1 preintegration complex to the nucleus. Formation of HIV-1 DNA occurs already within the preintegration complex and can even be formed within the intact virion itself. Complete HIV-1 DNA consists in several forms but, however, a crucial step in infection is the integration of viral DNA into the chromosomal host cell DNA. At this stage in the viral life cycle the cell is infected for life. Current anti-HIV compounds are directed against various stages in the HIV-1 life cycle. For instance, the nucleoside and non-nucleoside analogs are directed against reverse transcriptase, the enzyme that converts the viral RNA into DNA. In such a way virions released by HIV-infected cells are not infectious for other target cells, unless mutations in the reverse transcriptase occur that confer resistance to these classes of anti-HIV drugs. Another class of anti-HIV compounds that is part of all new triple anti-HIV therapy treatment regimens are the protease inhibitors. These compounds likely prevent the formation of complete virions by HIV-infected cells and thus are intended to prevent the spread of HIV-1 to new target cells. Herein is for example shown in figures 2, 4a and 4b that a pharmaceutical composition comprising a compound according to said general formula as provided by the invention provides anti-HIV activity as well, allowing for a novel antiviral therapy provided by the invention.

The invention furthermore provides use according to the invention wherein said treatment additionally comprises treatment with another pharmaceutical composition. For example, combinatorial therapy to treat virus infections, as is often the case when HIV-infected individuals are treated is now provided wherein said other pharmaceutical composition at least comprises an antiviral agent, such as for example amantadine and rimandatine or another anti-influenza agents, acyclovir, gangcyclovir or related agent, foscarnet or other anti-herpesvirus agent, ribavirine or a antiretroviral agent, or an antiviral agent as provided by the invention. Of course, combination therapies including an anti-viral agent according to the invention, and additionally comprising more than one additional anti-viral agent, such as combinations of said anti-viral agent as provided by the invention with nucleoside analogue reverse transcriptase inhibitors and/or with non-nucleoside reverse transcriptase inhibitors and/or with nucleotide analogue reverse transcriptase inhibitors and/or with protease inhibitors is provided as well, also since an anti-viral agent as provided by the invention is particularly effective against otherwise drug-resistant viruses.

Additionally, the invention provides use according to the invention wherein said treatment additionally comprises treatment of inflammatory responses, such as such as oedema, fever, algesia, neuromuscular pain, headache, cancer or arthritic pain, viral-infection-related or -associated dementia's, or other bodily ailments.

Furthermore, the invention provides a pharmaceutical composition intended and adapted for anti-viral therapy comprising a compound of said general formula or functional equivalent thereof. Preferably, said pharmaceutical composition intended and adapted for anti-viral therapy comprises a compound of said general formula wherein R or R' are as defined above. Preferably, an anti-viral agent as provided by the invention comprises 2 acetoxythioanisole, 2-(trifluoro-methylacetoxy)thioanisole, 2-(α chloroacetoxy)thioanisole, 2-(αbromoacetoxy)thioanisole, 2-acetoxyphenylbenzyl sulphide, 2-acetoxyphenzyl-2-phenylethyl sulphide, 2-acetoxyphenylethyl sulphide, 2-acetoxyphenylpropyl sulphide. 2-acetoxyphenyl-butyl sulphide, 2-acetoxyphenylpentyl sulphide, 2-acetoxy-phenylhexyl sulphide, 2-acetoxyphenylheptyl sulphide, 2-acetoxyphenyl-2-butoxyethyl sulphide, 2-acetoxyphenyl-2-trans-heptenyl sulphide. 2-acetoxyphenylhept-2-ynyl sulphide, 2-acetoxyphenylbut-2-ynyl sulphide, 2-acetoxyphenylprop-2-ynyl sulphide, or o-(acetoxy-phenyl)hept-2-ynyl sulphide (APHS), or a pharmaceutically acceptable salt or hydrate thereof. Suitable pharmaceutically acceptable salts are well known to those skilled in the art and include basic salts of inorganic and organic acids, such as hydrochloric acid, hydrobromic acid, sulphonic acid, phosphoric acid, methane sulphonic acid, ethane sulphonic acid, acetic acid, malic acid, tartaric acid, maleic acid, benzoic acid, salicylic acid, phenylacetic acid and mandelic acid. In addition, pharmaceutically acceptable salts of a compound according to said general formula may also be formed with a pharmaceutically acceptable cation, for instance, if a substituent group comprises a carboxy moiety. Suitable pharmaceutically acceptable cations are well known in the art and include alkaline, alkaline earth ammonium and quaternary ammonium cations.

In addition, the invention provides a pharmaceutical composition intended and adapted for anti-viral therapy comprising a compound of said general formula or functional equivalent thereof said composition at least combined, preferably mixed with a pharmaceutical composition that at least comprises another antiviral agent, such as for example amantadine and rimandatine or another anti-influenza agents, acyclovir, gangcyclovir or related agent, foscarnet or other anti-herpesvirus agent, ribavirine or a antiretroviral agent, or an antiviral agent as provided by the invention. Such a composition as provided by the invention can advantageously be used in combinatorial anti-viral therapy.

The invention also provides a method to treat a viral infection of an animal comprising administering to said animal an anti-viral agent according to the invention or subjecting said animal to treatment with an anti-viral agent according the invention. An anti-viral agent comprising a compound according to said general formula, a pharmaceuticaly acceptable salt thereof and a pharmaceutical composition incorporating such, may be conveniently administered by any of the routes conventionally used for drug administration, e.g., orally, topically, parenterally, or by inhalation. A compound according to said general formula may be administered in conventional dosage forms prepared by combining a compound according to said general formula with a standard pharmaceutical carrier according to conventional procedures.

An anti-viral agent comprising a compound according to said general formula may be administered parenterally, i.e., by intravenous, intramuscular, subcutaneous, intranasal, intrarectal, intravaginal or intraperitoneal administration. Subcutaneous and intramuscular forms of parenteral administration are generally preferred. Appropriate dosage forms and dosage regimes for such administration may be prepared by conventional techniques or arrived at by dose finding studies. Compounds may also be administered by inhalation e.g., intranasal and oral inhalation administration. Appropriate dosage forms or regimes for such administration, such as aerosol formulation or metered dose inhaler may be prepared by conventional techniques well known to those having ordinary skill in this art.

An anti-viral agent of the present invention may also be administered in combination with a known, second therapeutically active compound or composition. These procedures may involve mixing, granulating and compressing or dissolving the ingredients as appropriate to the desired preparation. It will be appreciated that the form and character of the pharmaceutically acceptable carrier or diluent is dictated by the amount of active ingredient with which it is to be combined, the route of administration and other well known variable. The carrier(s) must be "acceptable" in the sense of being compatible with the other ingredients of the formulation and not deleterious to the recipient thereof.

In particular, the invention provides a method to treat a viral infection comprising administering to said animal an anti-viral agent according to the invention or subjecting said animal to treatment with said agent wherein said viral infection is a retroviral infection. In particular, the invention provides a method to treat a viral infection wherein said animal is human. In the case of viral infections it is considered especially advantageous to combine treatment of a viral infection with an anti-viral agent according to the invention with treatment with at least one other anti-viral agent, thereby greatly enhancing the possible number of combinations that can be used to for example treat patients with retroviral infections such as AIDS or AIDS-related infections, thereby enhancing therapeutic possibilities for combinatorial or highly active antiviral therapy (HAART), especially under those circumstances wherein viral isolates may emerge or are already present in the patient that are otherwise at least partly resistant to other viral drugs such as a reverse transcriptase inhibitor or protease inhibitor.

### Material and methods

### 1. Materials and methods related to cells

### 1.a. Isolation and culture of peripheral blood mononuclear cells

Peripheral blood mononuclear cell (PBMC) fractions are isolated from heparinised blood from HIV-1-, HIV-2- and hepatitis B- seronegative donors (Blood-bank, Utrecht, the Netherlands) by Ficoll-Isopaque gradient separation. Cells are washed twice, stimulated with 4 µg/ml phytohemagglutinin (PHA), and cultured in RPMI-1640 medium supplemented with 5 mM Hepes, 19 mM sodium bicarbonate, 10 µg/ml gentamicin, and 10% heat-inactivated fetal calf serum at a concentration of 1 x 10⁶ cells/ml.

### 1.b. Isolation and culture of monocyte-derived macrophages (MDM)

PBMC are isolated from heparinized blood from HIV-1-, HIV-2-, and hepatitis B-seronegative donors and obtained on Ficoll-Hypaque density gradients. Cells are washed twice and monocytes are purified by countercurrent centrifugal elutriation. Cells are >98% monocytes by criteria of cell morphology on May-Grünwald-Giemsa-stained cytosmears and by nonspecific esterase staining using alpha-naphtylacetate (Sigma Chemical Co., St. Louis, MO) as substrate. Monocytes are cultured in suspension at a concentration of 2 x 10⁶ cells/ml in Teflon flasks (Nalgene, Rochester, NY) in Iscove's modified Dulbeco's medium (IMDM) with 10% heat-inactivated human AB serum negative for anti-HIV antibodies, 10 mg/ml gentamicin, and 10 mg/ml ciprofloxacin (Sigma) for 7 days.

### 1.c. Peripheral Blood Lymphocyte isolation

PBMC fractions are isolated from heparinized blood from HIV-1-, HIV-2- and hepatitis B- seronegative donors (Blood-bank, Utrecht, the Netherlands) by Ficoll-Isopaque gradient separation. After the cells are washed twice monocytes are allowed to adhere on fibronectin-coated flasks before the PBL fraction is harvested. The PBL fractions collected are of >85% purity as determined by May-Grünwald-Giemsa-staining. Isolated PBL are stimulated to proliferate for 3 days with 4 µg/ml phytohemagglutinin (PHA; Sigma). PBL are cultured in RPMI-1640 (Life Technologies Ltd.) medium supplemented with 10% heat inactivated fetal calf serum (LifeTechnologies Ltd.) and 10 mg/ml gentamicin (Life Technologies Ltd.). After PHA stimulation the cells are cultured in medium containing 10 U/ml human recombinant IL-2 (Boehringer) until use. Viability is >95% at the point of the initiation of the experiment as determined by trypan-blue exclusion.

### 1.d. Determination of cell viability using the MTT assay

Cell viability is assessed by the MTT assay. In short, cells are incubated with MTT 3-(4,5-dimethylthiazol-2-yl)-2,5-diphenyltetrazollumbromide (Sigma) for 2 hours at 37 °C. During this time viable cells convert MTT into water forming insoluble formazan dyes. Afterwards crystals are solubilised with a solution containing isopropanol. The OD of the supernatant is measured at 550 nm.

### 1.e. Determination of cell viability using the WST-1 assay

Cell viability is assessed with WST-1 assay. Cells are incubated with the tetrazolium salt WST-1 (4-[3-(4-lodophenyl)-2-(4-nitrophenyl)-2H-5-tetrazolio]-1,3-benzene disulfonate) for 2 hours at 37 °C. During this time viable cells convert WST-1 into water soluble formazan dyes. The OD of the supernatant is measured at 550 nm.

### 2. Materials and methods related to virus infection

### 2.a. Preparation of viral stocks

HIV-1 strain Ba-L is grown to high titer in MDM. The 50% tissue culture infectious dose (TCID₅₀) of the virus stock is determined by endpoint dilution with serial fourfold dilutions in a 96-well microtiter plate on MDM. HIV-1 strain AT is grown to high titer in PHA-stimulated PBMC. The TCID₅₀ of the virus stock is determined by endpoint dilution with serial fourfold dilutions in a 96-well microtiter plate on PBMC.

### 2.b. HIV-1 infection of cells (as determined by ELISA)

MDM, and PBMC, were incubated with HIV at a multiplicity of infection of 0.02, and 0.006 or 0.001, respectively. After two hours cells are washed to remove unbound virus and cultured for 4 to 7 days in different concentrations of the drug under investigation. On day 4, 5 and 7, 0.01 ml of supernatant is removed from the culture and virus in culture supernatant is inactivated in a final concentration of 0.05% empigen (Calbiochem-Novabiochem Co., La Jolla, CA). The presence of HIV-1 in the inactivated supernatant is monitored by checking for the p24-core antigen using the enzyme-linked immunosorbent assay (ELISA) system of John Moore.

### 3. Detection assays to test for the action of the drugs in the HIV life cycle

### 3.a. HIV-1 infection of cells transfected with different chemokine receptors to study the effect on HIV-1 entry into the cell

The cell line HOS-CD4 and the cell lines that are derived from HOS-CD4, namely HOS-CD4-CCR2b, HOS-CD4-CCR3, HOS-CD4-CCR5 and HOS-CD4-CXCR4 are obtainable through the AIDS Research and Reference Reagent Program. These cell lines express the chemokine receptors that are used by the different HIV-1 strains and are used for investigation of the effect of compounds of above identified general formula and related compounds on their antiviral effect on the different HIV-1 strains and on modulation of these chemokine receptors. In short, cells are infected with an appropriate viral strain and the anti-viral effect of said compounds is measured by HIV p24 antigen ELISA. Furthermore, the cells are incubated with a monoclonal antibody directed against the chemokine receptor studied. Then the cells will be ished twice with phosphate-buffered saline and analyzed on a FACStar flow cytometer (Beckton Dickinson and Co., Mountainview, CA).

### 3. b. RNA PCR detection of HIV-1 infection to study the effect on the transcriptional level

The HIV-infected cells are lysed in 1 ml TRIzol (Life Technologies Gaithersburg, MD) and RNA is isolated according to the manufacturer's guidelines. Total RNA is dissolved in diethylpyrocarbonate (DEPC)-treated water and 1 mg of RNA is used for the synthesis of complementary DNA. The RNA is previously heated for 5 minutes at 70°C, chilled on ice and added to a mixture containing 1x reverse transcriptase (RT) buffer (Promega, Madison, WI), 200 U of reverse transcriptase, 0.1 M dithiothreitol (DTT, Gibco, Grand Island, NY), 2.5 mM deoxynucleotidetriphosphate (dNTP's, Boehringer Mannheim, Indianapolis, IN), 80 U random hexamer oligonucleotides (Boehringer Mannheim) and 10 U RNAsin (Promega). The complete mixture is now incubated for 60 minutes at 37°C and then heated for 5 minutes at 90°C. The final reaction volume is diluted 1:8 by adding distilled water. Amplification of the cDNA is accomplished using one primer biotinylated on the 5' terminal nucleotide to facilitate later capture using streptavidin. To the PCR reaction mixture the following components are added: 0.25 mM dNTP mix (Boehringer Mannheim), 1 x PCR buffer (50 mM KCl, 10 mM Tris-HCl, 1.5 mM MgCl₂; Promega), 0.2 mM of the biotinylated HIV-1 tat/rev sense primer 5' GGC TTA GGC ATC TCC TAT GGC 3' or GAPDH sense primer 5' CCA TGG AGA AGG CTG GGG 3' and the antisense HIV-1 tat/rev primer 5' TGT CGG GTC CCC TCG TTG CTG G 3' or the antisense GAPDH primer 5' CAA ACT TGT CAT GGA TGA CC 3', 5 ml cDNA and 1 U Taq polymerase (Promega). Denaturation, annealing, and elongation temperatures for PCR are 94°C, 60°C, and 72°C for 1, 1, and 2 min each, using a DNA thermal cycler (Perkin-Elmer, Norwalk, CT). Negative controls are included in each assay to confirm that none of the reagents are contaminated with cDNA or previous PCR products. PCR is also performed on RNA samples to exclude genomic DNA contamination. To confirm single band product positive reactions are subjected to 40 cycles amplification and electrophoresis followed by ethidium bromide staining. Then, for semi-quantification every primer pair is tested at different cycle numbers to determine the linear range. GAPDH mRNA levels are high and 25 cycles is enough to measure the PCR product in its linear range, whereas HIV-1 tat/rev cDNA is subjected to 38 cycles to be in the linear range, when needed. Aliquots of 5 ml of the biotinylated PCR product are semi-quantitatively analyzed using a fluorescent digoxigenin detection ELISA kit (Boehringer Mannheim) according to manufacturer's protocol. In short, the biotinylated strand of denatured PCR product is captured by immobilized streptavidin. Then, a digoxigenin labeled probe (the probe for HIV-1 tat/rev is 5' CTT TGA TAG AGA AAC TTG ATG AGT CTG 3' and the probe for GAPDH is 5' CTG CAC CAC CAA CTG CTT AGC 3') is added followed by an alkaline phosphatase labeled antibody against digoxigenin. After addition of the substrate fluorescence is measured in relative fluorescence units (RFU) in a fluorescence multi-well plate reader (Perseptive biosystems, Framingham, MA) at excitation 450 nm/emission 550 nm. All data are normalized against GAPDH mRNA levels, which is used as an internal standard.

### 3.c. DNA PCR detection of HIV-1 infection to study the effect on the earliest processes of proviral DNA formation

Trizol reagent is used for DNA isolation according to the manufacturer's protocol. In short, DNA and RNA of cell samples in trizol are isolated by chloroform. DNA is precipitated from the lower chloroform phase by 100% ethanol and the sedimented DNA is ished twice in 0.1M sodium citrate in 10% ethanol. The pellet is reconstituted in water and checked for purity by measuring the OD260/280 ratio. The earliest processes of proviral DNA formation is analyzed by checking for the formation of the HIV R/U5 product indicating that the process of reverse transcription has taken place. The R/U5 primer pair flanks sequences within the first region of viral DNA synthesized as a result of reverse transcription, this first fragment of DNA is referred to as strong-stop minus DNA. The primer set which we use detects the early steps in reverse transcription and determines whether any viral DNA is synthesized in infected cells in the presence of APHS and derivates. The method and conditions of the PCR reaction are essentially the same as described in section 3.a. The R/U5 primer pairs (Zack et al, 1990): sense 5'-GGCTAACTAGGGAACCCACTG-3' and antisense 5'-TGTGTGCCCGTCTGTTGTGTG-3' (5' end biotinylated) result in a 132bp fragment. The digoxigenin-labeled probe 5'-TGTGTGCCCGTCTGTTGTGTG-3' is used to quantify the fragment. PCR amplification conditions are denaturation at 94°C for 5 min followed by 38 cycles of denaturation at 94°C for 1 min, annealing at 60°C for 1 min and extension at 72°C for 2 mins. The DNA product is finally extended at 72°C for 10 mins. 5 ml of the amplified product is quantified using the digoxigenin-labeled probe, by means of a DIG-detection ELISA (Boehringer-Mannheim, Mannheim, Germany).

### 3.d. HIV-LTR driven luciferase gene expression to study the effect on HIV promoter actiuity

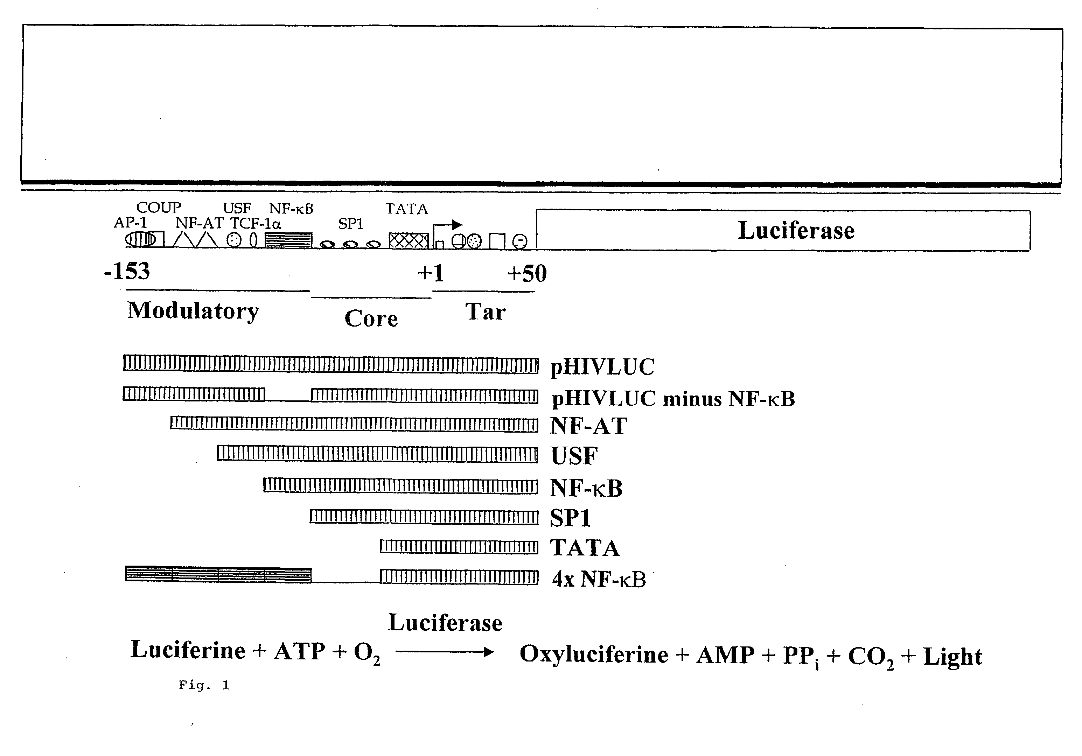

pHIV-CAT constructs are obtained from the NIH AIDS Research and Reference Reagent Program (National Institute of Allergy and Infectious diseases, Rockville, MD, USA). The HIV-CAT plasmids contained HindIII and BamHI restriction sites flanking the CAT gene. The HIV-long terminal repeat (LTR) sequence is found upstream this gene. The CAT gene is excised out of the plasmid and the luciferase (LUC) gene contained in a pGL3-basic vector (provided by Promega, Madison, USA) is obtained after HindIII and BamHI digestion. The LUC gene is then ligated into the empty HIV vectors, yielding HIV-LUC plasmids, with LTR-driven luciferase activity. The basic plasmid that does not contain any binding sites for eukaryotic transcription factors is pCD54 which only contains the 3' HIV-1 LTR region containing the TATA box and the TAR (where to HIV-1 tat can bind) region downstream of the LUC gene. In addition, the following plasmids are available: p3NF-kB wich contains 3 NF-kB binding sites downstream of pCD54; pCD52 which contains one binding site for SP1 downstream of pCD54; pCD23 which contains 3 SP1 binding sites and two NF-kB binding sites downstream of pCD54; pCD16 which contains one USF, one TCF-1a, two NF-kB, and three SP-1 binding sites downstream of pCD54; pCD7 which contains one NF-AT, one USF, one TCF-1a, two NF-kB, and three SP-1 binding sites downstream of pCD54; pHIV-LUC which contains the complete HIV-1 LTR region downstream of pCD54. The HIV-1 LTR consists of one AP-1 COUP, one NF-AT, one USF, one TCF-1a, two NF-kB, and three SP-1 binding sites. Figure 1 shows the collection of plasmids that are available.
E.coli DH5aF' that are made competent with CaCl₂ and are subsequently transformed with the pHIV-LUC vector and the other plasmids that are described above. The plasmids are isolated from these transformants after overnight incubation.

Cells (5 x 10⁶ cells/ml) are transfected by electroporation with 1 mg of a plasmid expressing the LUC reporter gene, under the control of the HIV-LTR. In addition to this plasmid the cells are co-transfected with 1 mg tat plasmid as an extra transcription stimulus and 1 mg β-gal plasmid as control for transfection efficiency After transfection, the cells are incubated at 37°C for 2 hours in medium containing 10% FCS and 10 mg/ml gentamicin. The transfected cells are subsequently incubated with various concentrations of the drug under investigation and then stimulated by 20 ng/ml phorbol 12-myristate 13-acetate (for PBMC and PBL) or 10mM N-acetyl-L-cysteine (for MDM). 16 hours after stimulation and compound incubation, firefly luciferase activity is measured employing the single-luciferase™ reporter assay system (Promega, Madison, USA). β-galactosidase activity is measured 16 hours after stimulation and compound addition. The amount of activity correlates to the light emission measured by LUMAC Biocounter M2500 at 562 nm. Cells stimulated in the absence of the drug under investigation serve as control cells.

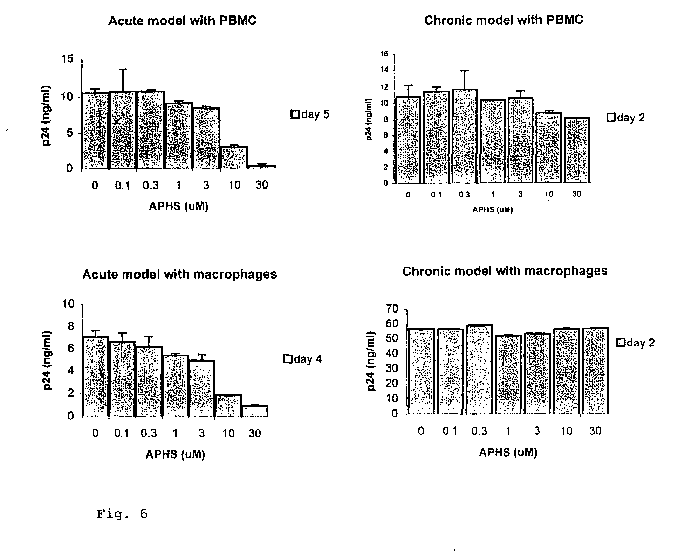

### Description of experiment with figure 6.

(A, B) Peripheral blood mononuclear cells (PBMC) were isolated from heparinized blood from HIV-1-, HIV-2-, and hepatitis B-seronegative donors and obtained on Ficoll-Hypaque density gradients. Cells were washed twice, stimulated with 5 µg/ml phytohemagglutinin (PHA), and cultured m RPMI-1640 medium supplemented with 5 mM Hepes, 19 mM sodium bicarbonate, 10 µg/mL gentamicin, and 10% heat-inactivated fetal calf serum (PBMC culture medium) at a concentration of 2 x 10⁶ cells/ml. After 3 days of incubation stimulated PBMC were recovered from the flasks and infected for 2 hours with HIV-1_{Ba-L} at a multiplicity of infection of 0.01. Afterwards, HIV-infected and mock-infected PBMC were washed twice to remove unbound virus. (A) PBMC were cultured at a density of 6 x 10⁵ cells/well with different concentrations of APHS and after 5 days of incubation samples of culture supernatant were collected. (B) PBMC were cultured in suspension at a concentration of 2 x 10⁶ cells/ml in Teflon flasks in PBMC culture medium. After 5 days of incubation PBMC were recovered from Teflon flasks, washed twice to remove unbound virus and cultured at a density of 6 x 10⁵ PBMC / well with different concentrations of APHS. After 2 days of incubation samples of culture supernatant were collected. p24-core antigen production was quantified using the enzyme-linked immunosorbent assay (ELISA) system of John Moore. While APHS does not inhibit HIV-1 production of chronically infected PBMCs (B), APHS concentrations above 3µM inhibit HIV-1_{Ba-L} replication and 30 µM APHS inhibits HIV-1Ba-L replication even by 100% (A). (C, D) PBMC were isolated from heparinized blood from HIV-1-, HIV-2-, and hepatitis B-seronegative donors and obtained on Ficoll-Hypaque density gradients. PBMC were washed twice and monocytes were purified by countercurrent centrifugal elutriation. Cells were >98% monocytes by criteria of cell morphology on May-Grünwald-Giemsa-stained cytosmears and by nonspecific esterase staining using alpha-naphtylacetate as substrate. Monocytes were cultured in suspension at a concentration of 2 x 10⁶ cells/ml in Teflon flasks in Iscove's modified Dulbeco's medium (IMDM) with 10% heat-inactivated human AB serum negative for anti-viral antibodies, 10 µg/ml gentamicin, and 10 µg/ml ciprofloxacin (MDM medium). After 7 days of incubation non-adherent monocyte-derived macrophages (MDM) were recovered from the Teflon flasks, washed and infected with HIV-1_{Ba-L} at a multiplicity of infection of 0.02 for two hours. Afterwards, HIV-infected and mock-infected MDM's were washed twice to remove unbound virus. (C) MDM were cultured cultured at a density of 3 x 10⁵ cells/well with different concentrations of APHS and after 4 days of incubation samples of culture supernatant were collected. (D) MDM were cultured in suspension at a concentration of 2 x 10⁶ cells/ml in Teflon flasks in MDM medium. After 5 days of incubation MDM were recovered from Teflon flasks, washed twice to remove unbound virus and cultured at a density of 3 x 10⁵ cells/well with different concentrations of APHS. After 2 days of incubation samples of culture supernatant were collected. p24-core antigen production was quantified using the enzyme-linked immunosorbent assay (ELISA) system of John Moore. While APHS does not inhibit HIV-1 production by chronically infected MDM (D), concentrations above 3 :M APHS inhibit p24 production by acutely infected MDM. 30:M APHS inhibits HIV-1 replication by 88% (C). The results shown in A-D are representative of three independent PBMC donors. These results suggest that APHS inhibits the early steps of the HIV-1 life cycle and that it is for instance not a protease inhibitor.

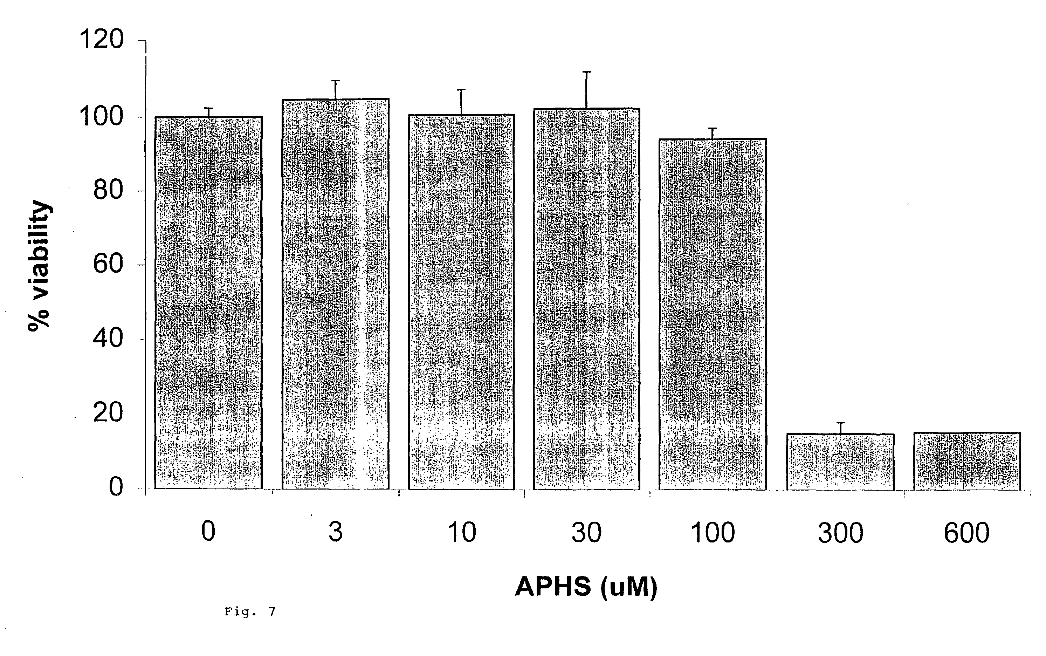

### Description of experiment with figure 7

Monocyte-derived macrophages (MDM) were obtained like described above. MDM were then washed twice and cultured for 4 to 7 days in different concentrations of APHS. After a 4 days incubation period cellular viability was assessed by MTT cytotoxicity assay where viable cells convert MTT into a colored formazan dye that can be measured spectrophotometrically.

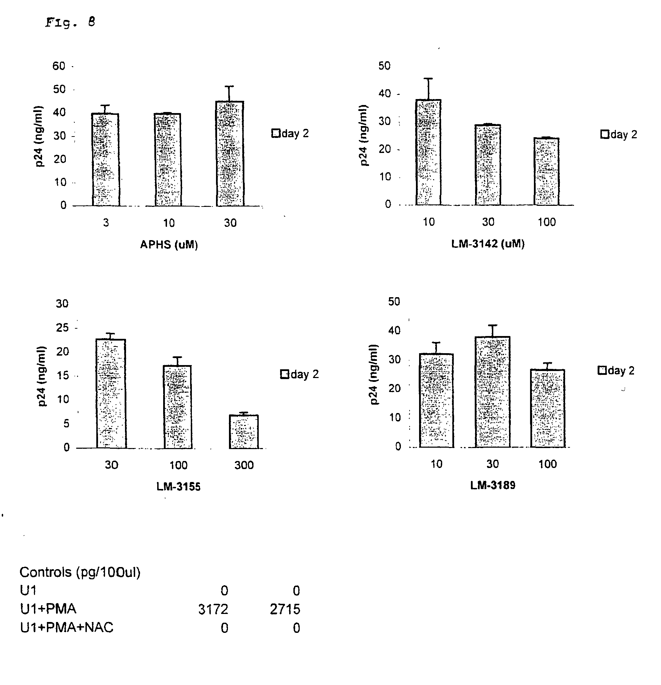

### Description of experiment with figure 8

U1 cells, chronically infected U937 cells that harbour two copies of the HIV-1IIIb strain genome in their cellular genome and don't produce viral particles, were stimulated with 100ng/ml phorbol 12-myristate 13-acetate (PMA) for 30 min. PMA treatment of U1 cells is known to trigger transcription of the HIV-1 genome and therefore the production of infectious particles. Afterwards cells were washed and cultured at a density of 1 x 10⁶ cells/well with different concentrations of APHS and related compounds (LM-3142, LM-3155 and LM-3189). As a negative control some cells were incubated with 30:M N-acetyl-L-cysteine (NAC) which is known to block PMA effect (data not shown).

Furthermore, APHS activity was tested with drug-resistant HIV strains (Table 2). Surprisingly, the inhibitory activity against otherwise drug resistant strains was high, sometimes even two- to four-fold higher than the activity against a sensitive strain.

**Table 1.**

| Antiretroviral agents (approved or in advanced development). |
|---|
| Nucleoside analogue reverse transcriptase inhibitors |
| Zidovudine (ZDV, AZT) |
| Didanosine (ddl) |
| Zalcitabine (ddC) |
| Stavudine (d4T) |
| Lamivudine (3TC) |
| Abacavir (1592U89) |

| Non-nucleoside reverse transcriptase inhibitors |
|---|
| Nevirapine |
| Delavirdine |
| Efavirenz (DMP-266) |

| Nucleotide analogue reverse transcriptase inhibitors |
|---|
| Adefovir dipivoxil |

| Protease inhibitors |
|---|
| Saquinavir |
| Ritonavir |
| Indinavir |
| Nelfinavir |
| Amprenavir (141W94, VX-478) |

**Table 2**

| IC50 values of APHS for drug-resistant HIV-1 strains | | |
|---|---|---|
| Virus | Resistance | IC50 of APHS (in M) |
| HXB2* | - | 12.6 +/- 2.3 |
| 41 + 215Y** | AZT | 5.1 +/- 1.9 |
| 184V*** | 3TC | 4.5 +/- 1.1 |
| 3096**** | High***** | 4.1 +/- 1.0 |
| 4602****** | Ritonavir | 5.2 |

| | | |
|---|---|---|
| HXB2 is the molecular clone of the first laboratory HIV-1 isolate. The genes that contain the drug-resistance mutations are excised from the clinical isolates and cloned into the genetic background of HXB2. Thus all tested HIV-1 strains have the same genetic background. | | |
| ** 41 + 215Y is a AZT-resistant HIV-1 strain (1). | | |
| *** 184V is a 3TC-resistant HIV-1 strain as described by Schuurman et al (2). | | |
| **** Strain 3096 is a RT inhibitor resistant HIV-1 strain as described by de Jong et al (3). This strain contains an insertion of two amino acids between codons 68 and 69 of RT as well as an amino-acid change at codon 67. | | |
| ***** Phenotypic resistance analysis showed high levels of resistance to zidovudine, lamivudine and stavudine (in all patients tested) and moderate levels of resistance to didanosine and zalcitabine (in two patients). | | |
| ****** Strain 4602 is a HIV protease resistant HIV-1 strain as described by Nijhuis et al. It contains the following mutations: 36I, 54V, 71V, and 77M (4). | | |

**Table 3.**

| IC50 and toxicity determinations of an anti-viral as provided herein. Side group references are as in figure 9. | | | | | | | | | | |
|---|---|---|---|---|---|---|---|---|---|---|
| | X | Y | Z | A | B | D | Y | E | IC₅₀* not tox | |
| APHS | O | S | H | C | O | CH₃ | S | CH₂C C(CH₂)₃CH₃ | 10 | 30 |
| BPHS | S | O | H | CH₂ | _ | C C(CH₂)₃CH₃ | O | (CH₂)₃CH₃ | 40 | 30 |
| c1 | O | S | H | C₂H₅ | _ | _ | S | CH₂C C(CH₂)₃CH₃ | 40 | 30 |
| c2 | O | S | H | C | O | CH₃ | S | (CH₂)₅CO₂H | 15 | 30 |
| c3 | O | S | H | C | O | CH₃ | S | (CH₂)₃O(CH₂)₂CH₃ | 0.3 | 30 |
| c4 | O | S | H | H | _ | _ | S | (CH₂)₅CO₂H | 30 | 10 |
| c5 | O | S | H | H | _ | _ | S | (CH₂)₃O(CH₂)₂CH₃ | 10 | 10 |
| c7 | O | S | H | C | O | CF₃ | S | (CH₂)₃O(CH₂)₂CH₃ | 0.3 | 10 |

| | | | | | | | | | | |
|---|---|---|---|---|---|---|---|---|---|---|
| * all concentrations are in micromolar | | | | | | | | | | |

### References

1.Differences in resistance contribute more strongly to the evolution of zidovudine resistance in HIV-1 infected patients than differences m replication capacity.
2. Schuurman R, Nijhuis M, van Leeuwen R, Schipper P, de Jong D, Collis P, Danner SA, Mulder J, Loveday C, Christopherson C, Kwok S, Sninsky J, Boucher CA. Rapid changes in human immunodeficiency virus type 1 RNA load and appearance of drug-resistant virus populations in persons treated with lamivudine (3TC) J. Infect. Dis. 1995;171:1411-1419.
3. de Jong JJ, Goudsmit J, Lukashov VV, Hillebrand ME, Baan E, Huismans R, Danner SA, ten Veen JH, de Wolf F, Jurriaans S. Insertion of two amino acids combined with changes in reverse transcriptase containing tyrosine-215 of HIV-1 resistant to multiple nucleoside analogs. AIDS 1999;13:75-80.
4.Nijhuis M, Schuurman R, de Jong D, Erickson J, Gustchina E, Albert J, Schipper P, Gulnik S, Boucher CA. Increased fitness of drug resistant HIV-1 protease as a result of acquisition of compensatory mutations during suboptimal therapy. AIDS 1999;13:2349-59.

### Figure legends

Fig. 1 - Plasmids that are used for studies on the effect of compounds on HIV promotor activity.
Fig. 2 - Peripheral blood mononuclear cells (PBMC) were isolated from heparinized blood from HIV-1-, HIV-2-, and hepatitis B-seronegative donors and obtained on Ficoll-Hypaque density gradients. PBMC were washed twice and monocytes were purified by countercurrent centrifugal elutriation. Cells were >98% monocytes by criteria of cell morphology on May-Grünwald-Giemsa-stained cytosmears and by nonspecific esterase staining using alpha-naphtylacetate as substrate. Monocytes were cultured in suspension at a concentration of 2 x 10⁶ cells/ml in Teflon flasks in Iscove's modified Dulbeco's medium (IMDM) with 10% heat-inactivated human AB serum negative for anti-HIV antibodies, 10 mg/ml gentamicin, and 10 mg/ml ciprofloxacin. After 7 days of incubation non-adherent monocyte-derived macrophages (MDM) were recovered from the Teflon flasks, washed and infected with HIV-1_{Ba-L} at a multiplicity of infection of 0.02 for two hours. HIV-infected and mock-infected MDM's were washed twice to remove unbound virus and cultured for 4 to 7 days in different concentrations of APHS. After 4 and 5 days of incubation samples of culture supernatant were collected and p24-core antigen production was quantified using the enzyme-linked immunosorbent assay (ELISA) system of John Moore. Concentrations above 0.3 µM APHS inhibit p24 production 30 µM APHS inhibits HIV-1 replication by 88%.
Fig 3 - Monocyte-derived macrophages (MDM) were obtained like described in legend of Fig.2. MDM were then washed twice and cultured for 4 to 7 days in different concentrations of APHS. After a 4 days incubation period cellular viability was assessed by MTT cytotoxicity assay where viable cells convert MTT into a colored formazan dye that can be measured spectrophotometrically. None of the tested concentrations of APHS was found to be cytotoxic.

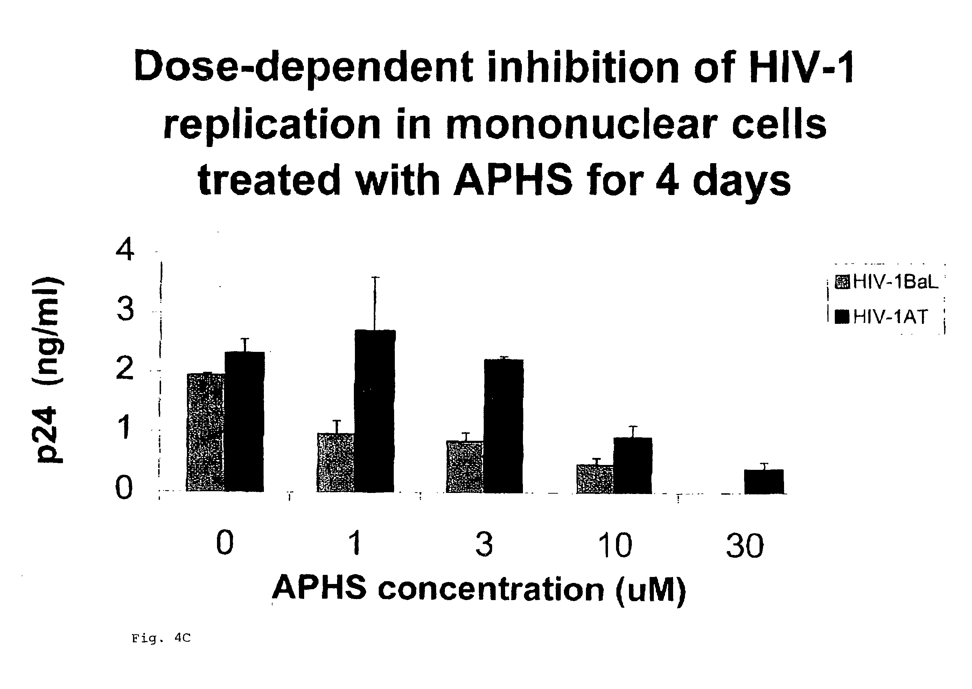

Fig 4 - Peripheral blood mononuclear cells (PBMC) were isolated from heparinized blood from HIV-1-, HIV-2-, and hepatitis B-seronegative donors and obtained on Ficoll-Hypaque density gradients. Cells were washed twice, stimulated with 5 mg/ml phytohemagglutinin (PHA), and cultured in RPMI-1640 medium supplemented with 5 mM Hepes, 19 mM sodium bicarbonate, 10 mg/mL gentamicin, and 10% heat-inactivated fetal calf serum at a concentration of 2 x 10⁶ cells/ml. After 3 days of incubation stimulated PBMC were recovered from the flasks and infected for 2 hours with (a) HIV-1_{AT} at a multiplicity of infection of 0.001, (b) HIV-1_{BaL} at a multiplicity of infection of 0.006 and (c) HIV-1_{BaL} or HIV-1_{AT} at a multiplicity of infection of 0.01 or 0.001, respectively. HIV-infected and mock-infected PBMC were washed twice to remove unbound virus and cultured for 4 to 7 days in different concentrations of APHS. After 4 and 5 days of incubation samples of culture supernatant were collected and p24-core antigen production was quantified using the enzyme-linked immunosorbent assay (ELISA) system of John Moore.
   Concentrations above 1 µM APHS inhibit HIV-1 production. 30 µM APHS inhibit HIV-1BaL replication by 100%. When infectivity is lower (Fig 4c), 1 µM APHS inhibit HIV-1_{BaL} production by at 50%.
Fig 5 - PBMC were obtained and stimulated as described in legend of Fig. 4. 3 days after incubation PBMC were recovered from the flasks, washed and cultured for 4 to 7 days in different concentrations of APHS. After a 5 days incubation period cellular viability was assessed by WST-1 cytotoxicity assay where viable cells convert WST-1 into a coloured formazan dye that can be measured spectrophotometrically. Concentrations at or above 100 µM were found to reduce viability.
Figure 6: Effect of APHS on HIV-1 _{Ba-L} replication in PBMC (A,B) or MDM (C,D) in an acute (A,C) or chronic (B,D) Model of HIV-1 infection.
Figure 7: Viability of monocyte-derived macrophage after APHS treatment.
Figure 8: Effect of APHS on HIV-1 production by U1 cells.
Figure 9: Reference figure for figure 9.
Figure 10: The 50% inhibitory concentration (IC₅₀) of the compounds APHS, LM-3177, LM-3189, LM-3142, LM-3155 and aspirin that suppresses HIV-1 replication in primary human peripheral blood mononuclear cells. The infection was performed as described with figure 6A. The IC50 values are depicted m micromolars. The letters X, Y, Z, A. B, D, E in the first row (upper line) refer to the same letters in the general formula of the compound in figure 9.
Figure 11: Selected chemical structures for letters X, Y, Z, A, B, D, E in the general formula of the compound in figure 9. Of particular interest are the combinations in these letters whereby Z = H, Y = S, E = CH₂CEC(CH₂)₃CH₃, X = O, B = -, D= -, and A is either CH₃, CH₂CH₃, CH₂CH₂CH₃, CH₂(CH₂)₂CH₃, CH₂(CH₂)₃CH₃, CH₂(CH₂)₄CH₃, CH₂(CH₂)₅CH₃, or CH₂(CH₂)₆CH₃ and the combinations in letters whereby Z = H, Y = S, E = C(CH₃)₃, X = O, A = C, B = O, and D = CH₃ and the combinations whereby Z = H, Y = S, E = C(CH₃)₃, X = -, A = C, B = O, and D = CH₃ and the combinations whereby Z = SC(CH₃)₃, Y = -, E= -, X = O, A = C, B = O, and D = CH₃ and the combinations whereby Z = SC(CH₃)₃, Y = -, E= -, X = -, A = C, B = O, and D = CH₃ and the combinations whereby Y = -, E = -, Z = SCH₂CEC(CH₂)₃CH₃, X = O, B = -, D= -, and A is either CH₃, CH₂CH₃, CH₂CH₂CH₃, CH₂(CH₂)₂CH₂, CH₂(CH₂)₃CH₃, CH₂(CH₂)₄CH₃, CH₂(CH₂)₅CH₃, or CH₂(CH₂)₆CH₃.

## Claims

1. Use of a compound of the general formula 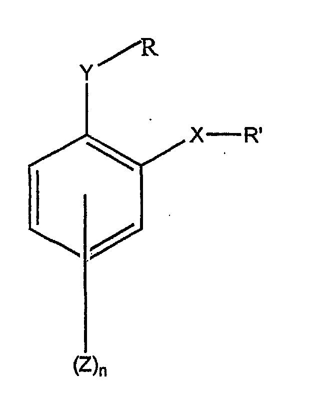 wherein X and Y are independently O, S, SO, SO₂, SO₃; wherein n is 0, 1, 2, 3, 4; wherein R, R' are independently H with the proviso that when R is H, R' is not H, a C₁-C₁₀, branched or unbranched, substituted or unsubstituted, saturated or (poly)unsaturated, (cyclo)alkyl, alkene, alkyn, (cyclo)aryl, aryl(cyclo)alkyl, (cyclo)alkylaryl, alkoxyaryl, alkoxyalkene, alkoxyalkyne, enyne, diene, diyne or alkoxyalkyl; and wherein R' is R; and wherein R or R' may contain ether linkages or carbonyl or thiocarbonyl functions attached to the ring structure such as ring-(C=O/S)-R/R' and Z is independently R, R', XR, XR', YR or YR' or a functional equivalent thereof for the production of a pharmaceutical composition for the treatment of a viral infection.

2. Use of a pharmaceutically acceptable salt or hydrate of a compound according to claim 1, for the production of a pharmaceutical composition for the treatment of a viral infection.

3. Use according to claim 1 or 2, wherein X and Y are O, S, SO₂.

4. Use according to any one of claims 1-3, wherein n is 1 or 2.

5. Use according to any one of claims 1-4, wherein R, R' are substituted with one or more of halogen or CF₃.

6. Use according to any one of claims 1-5 wherein R is selected from the group consisting of H, CH₃, CF₃, CH₂Cl, CH₂Br, CH₂CH₃, (CH₂)₂CH₃, (CH₂)₃CH₃, (CH₂)₄CH₃, (CH₂)₅CH₃, (CH₂)₆CH₃, CH(CH₃)₂, C(CH₃)₃, CH=C=CH₂, (CH₂)₂O(CH₂)₃CH₃, CH₂HC=CH(CH₂)₃CH₃, CH₂C≡C(CH₂)₃CH₃, CH₂C≡C(CH₂)₂CH₃, CH₂C≡CCH₂CH₃, CH₂C≡CCH₃ and CH₂C≡CH and isomers or homologues thereof.

7. Use according to any one of claims 1-6, wherein R' is R and selected from the group consisting of H, CH₃.

8. Use of a compound of the general formula 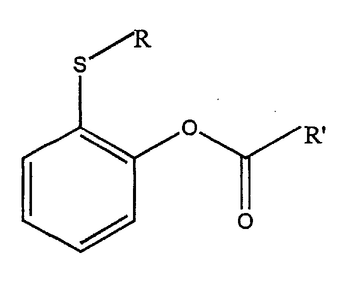 or a functional equivalent thereof for the production of a pharmaceutical composition for the treatment of a viral infection.

9. Use according to claim 8 wherein R is selected from the group consisting of H, CH₃, CH₂CH₃, (CH₂)₂CH₃, (CH₂)₃CH₃, (CH₂)₄CH₃, (CH₂)₅CH₃, (CH₂)₆CH₃, CH(CH₃)₂, C(CH₃)₃, CH=C=CH₂, (CH₂)₂O(CH₂)₃CH₃, CH₂HC=CH(CH₂)₃CH₃, CH₂C≡C(CH₂)₃CH₃, CH₂C≡C(CH₂)₂CH₃, CH₂C≡CCH₂CH₃, CH₂C≡CCH₃ and CH₂C≡CH and isomers or homologues thereof and R' is selected from the group consisting of H, CH₃, CF₃, CH₂Cl and CH₂Br.

10. Use according to any one of claims 1-9 wherein said viral infection comprises a retroviral infection.

11. Use according to claim 10 wherein said retroviral infection is caused by a retrovirus at least partly resistant against treatment with another antiviral agent.

12. Use according to any one of claims 1-11 wherein said treatment additionally comprises treatment with another pharmaceutical composition.

13. Use according to any one of claims 1-12 wherein said other pharmaceutical composition at least comprises an antiviral agent.

14. Use according to any one of claims 1-13 wherein said treatment additionally comprises treatment of inflammatory responses.

15. Kit-of-parts for use in the treatment of a viral infection comprising the compound of a formula as identified in any one of claims 1-14 or a pharmaceutically acceptable salt or hydrate thereof and a pharmaceutical composition that at least comprises another antiviral agent.

## Patentansprüche

1. Gebrauch einer Zusammensetzung der generellen Formel 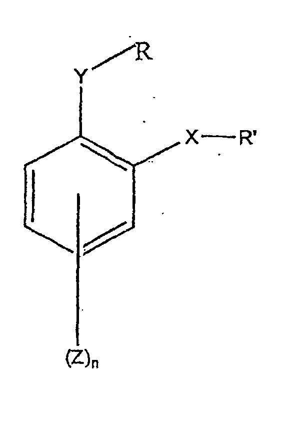 dabei sind X und Y unabhängig O, S, SO, SO₂, SO₃; dabei ist n 0, 1, 2, 3, 4; dabei sind R, R' unabhängig H, mit der Bedingung, dass wenn R H ist, R' nicht H ist, eine C₁-C₁₀, verzweigte oder unverzweigte, substituierte oder unsubstituierte, gesättigte oder (poly)ungesättigte, (Cyklo)Alkyl, Alken, Alkyn, (Cyklo)Aryl, Aryl(Cyklo)Alkyl, (Cyklo)Alkylaryl, Alkoxyaryl, Alkoxyalken, Alkoxyalkyn, Enyn, Dien, Diyn oder Alkoxyalkyl; und dabei ist R' R; und dabei können R oder R' Ether-Verbindungen enthalten oder Carbonyl oder Thiocarbonyl-Funktionen an die Ringstruktur angehängt haben wie eine Ring-(C=O/S)-R/R' und Z ist unabhängig R, R', XR, XR', YR oder YR' oder ein funktionelles Äquivalent davon zur Herstellung einer pharmazeutischen Zusammensetzung für die Behandlung einer Vireninfektion.

2. Gebrauch eines pharmazeutisch akzeptablen Salzes oder Hydrats einer Zusammensetzung gemäß Anspruch 1 für die Herstellung einer pharmazeutischen Zusammensetzung für die Behandlung einer Vireninfektion.

3. Gebrauch gemäß Anspruch 1 oder 2, dabei sind X und Y O, S, SO₂.

4. Gebrauch gemäß einem der Ansprüche 1 bis 3, dabei ist n 1 oder 2.

5. Gebrauch gemäß einem der Ansprüche 1 bis 4, dabei werden R, R' substituiert mit einem oder mehreren der Halogene oder CF₃.

6. Gebrauch gemäß einem der Ansprüche 1 bis 5, wobei R gewählt wird aus der Gruppe die besteht aus H, CH₃, CF₃, CH₂Cl, CH₂Br, CH₂CH₃, (CH₂)₂CH₃, (CH₂)₃CH₃, (CH₂)₄CH₃, (CH₂)₅CH₃, (CH₂)₆CH₃, CH(CH₃)₂, C(CH₃)₃, CH=C=CH₂, (CH₂)₂O(CH₂)₃CH₃, CH₂HC=CH(CH₂)₃CH₃, CH₂C≡C(CH₂)₃CH₃, CH₂C≡C(CH₂)₂CH₃, CH₂C≡CH₂CH₃, CH₂C≡CCH₃ und CH₂C≡CH und Isomeren und Homologen davon.

7. Gebrauch gemäß einer der Ansprüche 1 bis 6, dabei ist R' R und ausgewählt aus der Gruppe, die besteht aus H, CH₃.

8. Gebrauch einer Zusammensetzung der generellen Formel 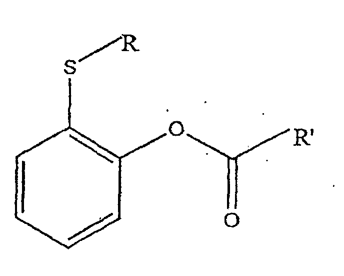 oder einem funktionellen Äquivalent davon für die Herstellung einer pharmazeutischen Zusammensetzung für die Behandlung einer Vireninfektion.

9. Gebrauch gemäß Anspruch 8, wobei R gewählt wird aus der Gruppe, die besteht aus H, CH₃, CH₂CH₃, (CH₂)₂CH₃, (CH₂)₃CH,₃, (CH₂)₄CH₃, (CH₂)₅CH₃, (CH₂)₆CH₃, CH(CH₃)₂, C(CH₃)₃, CH=C=CH₂, (CH₂)₂O(CH₂)₃CH₃, CH₂HC=CH(CH₂)₃CH₃, CH₂≡C(CH₂)₃CH₃, CH₂C≡C(CH₂)₂CH₃, CH₂C≡CCH₂CH₃, CH₂C≡CCH₃ und CH₂C≡CH sowie Isomeren und Homologen davon und R' wird ausgewählt aus der Gruppe, die besteht aus H, CH₃, CF₃, CH₂Cl und CH₂Br.

10. Gebrauch gemäß einem der Ansprüche 1 bis 9, wobei die genannte Vireninfektion eine retrovirale Infektion umfasst.

11. Gebrauch gemäß Anspruch 10, wobei die genannte retrovirale Infektion verursacht wird durch einen Retrovirus, das wenigstens teilweise resistent ist gegen eine Behandlung mit einem anderen Antivirenmittel.

12. Gebrauch gemäß einem der Anspruch 1 bis 11, wobei die Behandlung zusätzlich die Behandlung mit einer anderen pharmazeutischen Zusammensetzung umfasst.

13. Gebrauch gemäß einem der Ansprüche 1 bis 12, wobei die genannte andere pharmazeutische Zusammensetzung wenigstens ein Antivirenmittel umfasst.

14. Gebrauch gemäß einem der Ansprüche 1 bis 13, wobei die genannte Behandlung zusätzlich die Behandlung von entzündlichen Reaktionen umfasst.

15. Teile-Set zum Gebrauch bei der Behandlung einer Vireninfektion enthaltend die Zusammensetzung einer Formel gemäß einem der Ansprüche 1 bis 14 oder einem pharmazeutisch akzeptablen Salz oder Hydrats davon und eine pharmazeutische Zusammensetzung, die wenigstens ein anderes Antivirenmittel enthält.

## Revendications

1. Utilisation d'un composé selon la formule générale 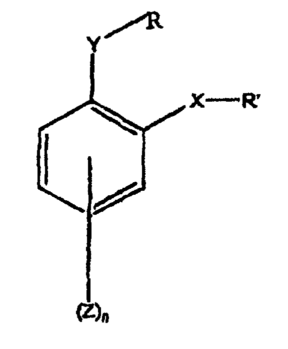 où X et Y sont indépendamment O, S, SO, SO₂, SO₃, où n vaut 0, 1, 2, 3, 4;
où R, R' sont indépendamment H à condition que : lorsque R est H, R' ne soit pas H, un (cyclo)alkyle, un alcène, un alcyne, un (cyclo)aryle, un aryl(cyclo)alkyle, un (cyclo)alkylaryle, un alcoxyaryle, un alcoxyalcène, un alcoxyalcyne, un ényne, un diène, un diyne ou un alcoxyalkyle ramifié en C₁ à C₁₀ ou non ramifié, substitué ou non substitué, saturé ou (poly)insaturé; et où R' est R; et où R ou R' peuvent contenir soit des liaisons, soit des fonctions carbonyle ou thiocarbonyle rattachées à la structure cyclique telle qu'un cycle (C=O/S)-R/R' et Z est indépendamment R, R', XR, XR', YR ou YR' ou un équivalent fonctionnel de celui-ci pour la production d'une composition pharmaceutique pour le traitement d'une infection virale.

2. Utilisation d'un sel pharmaceutiquement acceptable ou d'un hydrate d'un composé selon la revendication 1, pour la production d'une composition pharmaceutique destinée au traitement d'un infection virale.

3. Utilisation selon la revendication 1 ou 2, dans laquelle X et Y sont O, S, SO₂.

4. Utilisation selon l'une quelconque des revendications 1 à 3, où n vaut 1 ou 2.

5. Utilisation selon l'une quelconque des revendications 1 à 4, où R, R' sont substitués par un ou plusieurs d'un halogène ou de CF₃.

6. Utilisation selon l'une quelconque des revendications 1 à 5, dans laquelle R est choisi parmi le groupe constitué de H, CH₃, CF₃, CH₂Cl, CH₂Br, CH₂CH₃, (CH₂)₂CH₃, (CH₂)₃CH₃, (CH₂)₄CH₃, (CH₂)CH₃, (CH₂)₆CH₃, CH(CH₃)₂, C(CH₃)₃, CH=C=CH₂, (CH₂)₂O(CH₂)₃CH₃, CH₂HC=CH(CH₂)₃CH₃, CH₂C≡C(CH₂)₃CH₃, CH₂C≡C(CH₂)₂CH₃, CH₂C≡CCH₂CH₃, CH₂C≡CCH₃ et CH₂C≡CH et des isomères ou des homologues de ceux-ci.

7. Utilisation selon l'une quelconque des revendications 1 à 6, dans laquelle R' représente R et est sélectionné parmi le groupe constitué de H, CH₃.

8. Utilisation d'un composé selon la formule générale 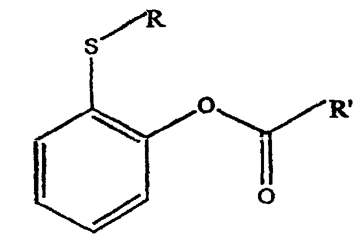 ou un équivalent fonctionnel de celui-ci pour la production d'une composition pharmaceutique en vue du traitement d'une infection virale.

9. Utilisation selon la revendication 8, dans laquelle R est sélectionné parmi le groupe constitué de H, CH₃, CH₂CH₃, (CH₂)₂CH₃, (CH₂)₃CH₃, (CH₂)₄CH₃, (CH₂)₅CH₃, (CH₂)₆CH₃, CH(CH₃)₂, C(CH₃)₃, CH=C=CH₂, (CH₂)₂O(CH₂)₃CH₃, CH₂HC=CH(CH₂)₃CH₃, CH₂C≡C(CH₂)₃CH₃, CH₂C≡C(CH₂)₂CH₃, CH₂C≡CCH₂CH₃, CH₂C≡CCH₃ et CH₂C≡CH et des isomères ou des homologues de ceux-ci et R' est choisi parmi le groupe constitué de H, CH₃, CF₃, CH₂Cl et CH₂Br.

10. Utilisation selon l'une quelconque des revendications 1 à 9, dans laquelle ladite infection virale comprend une infection retrovirale.

11. Utilisation selon la revendication 10, dans laquelle ladite infection rétrovirale est provoquée par un rétrovirus au moins partiellement résistant contre un traitement avec un autre agent antiviral.

12. Utilisation selon l'une quelconque des revendications 1 à 11, dans laquelle ledit traitement comprend en outre un traitement avec une autre composition pharmaceutique.

13. Utilisation selon l'une quelconque des revendications 1 à 12, dans laquelle ladite autre composition pharmaceutique comprend au moins un agent antiviral.

14. Utilisation selon l'une quelconque des revendications 1 à 13, dans laquelle ledit traitement comprend en outre un traitement des réponses inflammatoires.

15. Nécessaire de composants à utiliser dans le traitement d'une infection virale comprenant le composé selon une formule telle qu'identifiée dans l'une quelconque des revendications 1 à 14 ou un sel pharmaceutiquement acceptable ou un hydrate de celui-ci et une composition pharmaceutique qui comprend au moins un autre agent antiviral.
